# EUROPEAN PATENT SPECIFICATION

(11) **EP 1 714 134 B1**
(45) Date of publication and mention of the grant of the patent: **21.04.2010**
(21) Application number: 05702057.0
(22) Date of filing: 28.01.2005
(51) Int. Cl.: G01N 1/28, G01N 27/447, C12Q 1/68

(54) **A DIAGNOSTIC SYSTEM FOR CARRYING OUT A NUCLEIC ACID SEQUENCE AMPLIFICATION AND DETECTION PROCESS**
DIAGNOSESYSTEM ZUR DURCHFÜHRUNG EINES NUKLEINSÄURESEQUENZAMPLIFIKATIONS- UND -NACHWEISVERFAHRENS
SYSTEME DIAGNOSTIC SERVANT A REALISER UNE AMPLIFICATION DE SEQUENCES D'ACIDES NUCLEIQUES ET PROCEDE DE DETECTION

(30) Priority: 28.01.2004 GB 0401868
(43) Date of publication of application: 25.10.2006
(73) Proprietor: Norchip A/S, 3490 Klokkarstua (NO)
(72) Inventor: SCHONFELD, F., Inst. für Mikrotechnik Mainz GmbH, 55129 Mainz (DE); VON GERMAR, F., Inst. für Mikrotechnik Mainz GmbH, 55129 Mainz (DE); KARLSEN, Frank, N-3490 Klokkarstua (NO); LICHTENBERG, Jan, Inst. of Microtechnology, CH-2000 Neuchatel (CH); VERPOORTE, Sabeth, Inst. of Microtechnology, CH-2000 Neuchatel (CH)
(74) Representative: Setna, Rohan P.
(86) International application number: PCT/GB2005/000308
(87) International publication number: WO 2005/073691

(56) References cited:
- EP-A- 0 987 327
- WO-A-00/62931
- WO-A-2004/096443
- DE-A1- 19 700 364
- US-A- 5 229 297
- US-A1- 2002 045 246
- US-A1- 2003 138 941
- US-B1- 6 544 734
- JOON-HO KIM ET AL: "A disposable DNA sample preparation microfluidic chip for nucleic acid probe assay" PROCEEDINGS OF THE IEEE 15TH. ANNUAL INTERNATIONAL CONFERENCE ON MICROELECTRO MECHANICAL SYSTEMS. MEMS 2002. LAS VEGAS, NV, JAN. 20 - 24, 2002, IEEE INTERNATIONAL MICRO ELECTRO MECHANICAL SYSTEMS CONFERENCE, NEW YORK, NY : IEEE, US, vol. CONF. 15, 2002, pages 133-136, XP010577613 ISBN: 0-7803-7185-2
- PRINZ ET AL.: "Bacterial Chromosome extraction and isolation" LAB CHIP, vol. 2, 2002, pages 207-212, XP009047795
- SCHILLING E A ET AL: "CELL LYSIS AND PROTEIN EXTRACTION IN A MICROFLUIDIC DEVICE WITH DETECTION BY A FLUOROGENIC ENZYME ASSAY" ANALYTICAL CHEMISTRY, AMERICAN CHEMICAL SOCIETY. COLUMBUS, US, vol. 74, no. 8, 15 April 2002 (2002-04-15), pages 1798-1804, XP001115858 ISSN: 0003-2700
- WATERS L C ET AL: "MICROCHIP DEVICE FOR CELL LYSIS, MULTIPLEX PCR AMPLIFICATION, AND ELECTROPHORETIC SIZING" ANALYTICAL CHEMISTRY, AMERICAN CHEMICAL SOCIETY. COLUMBUS, US, vol. 70, no. 1, January 1998 (1998-01), pages 158-162, XP000733220 ISSN: 0003-2700
- ZHANG N ET AL: "AUTOMATED AND INTEGRATED SYSTEM FOR HIGH-THROUGHPUT DNA GENOTYPING DIRECTLY FROM BLOOD" ANALYTICAL CHEMISTRY, AMERICAN CHEMICAL SOCIETY. COLUMBUS, US, vol. 71, no. 6, 15 March 1999 (1999-03-15), pages 1138-1145, XP000831550 ISSN: 0003-2700

## Description

The present invention is concerned with nucleic acid (NA) extraction and, in particular, an integrated lab-on-a-chip diagnostic system for carrying out combined NA extraction and concentration. The system may be used to carry out a NA sequence amplification and detection process on a fluid sample containing cells.

There is considerable interest in the development of simplified assay systems for detection of biological molecules which allow an unskilled user to perform complex assay procedures without undue error. Moreover, there is a great deal of interest in the development of contained assay systems which require minimal handling of liquid reagents and which can be automated to allow the assay procedure to be performed with minimal intervention from the user, and preferably also miniaturized to provide a convenient system for point-of-care testing. This is particularly relevant in the healthcare field, especially diagnostics, where there is an increasing need for biological assay systems which can be efficiently and safely operated within the doctor's surgery, the clinic, the veterinary surgery or even in the patient's home or in the field.

Microfabricated "lab-on-a-chip" devices are an attractive option for carrying out contained biological reactions requiring minimal reagent handling by the user and also permit the use of small sample volumes, a significant advantage for biological reactions which require expensive reagents.

To achieve both purification and preconcentration, analytical chemists have generally resorted to some kind of extraction procedure. These methods involve removal of the analytes of interest from the sample matrix, or alternatively, removing all other species from the sample matrix to leave behind the analytes of interest. Extraction processes can involve transfer of species from one liquid phase to another, or the capture of species from a liquid phase onto a solid surface. In the former case, preconcentration of a species is generally not achieved, unless solvent is actively removed from the phase containing that species. In the latter case, however, preconcentration can be achieved, if (a) the available binding area is large enough to bind more molecules than are present in the solution in contact with the surface at any one time, and (b) species can be efficiently removed from the solid phase using only a small amount of eluent. Since preconcentration is an important aspect of the nucleic acid sample pre-treatment procedure, solid-phase extraction has been adopted. A well-established nucleic acid extraction method involving binding of DNA to silica particles in the presence of a chaotropic agent (see Boom et al, J. Clin. Microbiol. 1990, 28, 495-503). The present invention involves integration of a solid-phase extraction method for DNA into microfluidic devices.

In the present invention NA extraction and concentration may be combined.

By the term microfabricated device or system as used herein is meant any device manufactured using processes that are typically, but not exclusively, used for batch production of semiconductor microelectronic devices, and in recent years, for the production of semiconductor micromechanical devices. Such microfabrication technologies include, for example, epitaxial growth (eg vapour phase, liquid phase, molecular beam, metal organic chemical vapour deposition), lithography (eg photo-, electron beam-, x-ray, ion beam-), etching (eg chemical, gas phase, plasma), electrodeposition, sputtering, diffusion doping and ion implantation. Although non-crystalline materials such as glass may be used, microfabricated devices are typically formed on crystalline semiconductor substrates such as silicon or gallium arsenide, with the advantage that electronic circuitry may be integrated into the system by the use of conventional integrated circuit fabrication techniques. Combinations of a microfabricated component with one or more other elements such as a glass plate or a complementary microfabricated element are frequently used and intended to fall within the scope of the term microfabricated used herein. Also intended to fall within the scope of the term microfabricated are polymeric replicas made from, for example, a crystalline semiconductor substrate.

The isolation and purification of DNA and/or RNA from bacterial cells and virus particles is a key step in many areas of technology such as, for example, diagnostics, environmental monitoring, forensics and molecular biology research.

Microfabrication is an attractive construction method for producing devices for carrying out biological processes for which very small sample volumes are desirable, such as DNA sequence analysis and detection.

One such device, for carrying out a polymerase chain reaction (PCR) followed by a detection step is disclosed in US 7169601 B. Lamb wave pumps are used to transport DNA primers, polymerase reagents and nucleotide reagents from three separate storage chambers into a single reaction chamber as and when required to carry out a PCR process, with the temperature of the reaction chamber being cycled as required.

Another microfabricated device, for carrying out a chemical reaction step followed by an electrophoresis separation step, is disclosed in Analytical Chemistry 1994, 66,4127-4132. Etched structures in a silicon substrate covered by a glass plate provide a reaction chamber and connections to buffer, analyte, reagent and analyte waste reservoirs, as well as an electrophoresis column connected to a waste reservoir.

Nucleic acid sequence-based amplification (NASBA) is a primer-dependent technology that can be used for the continuous amplification of nucleic acids in a single mixture at one temperature (isothermal nucleic acid amplification method) and was one of the first RNA transcription-based amplification methods described. NASBA normally offers a simple and rapid alternative to PCR for nucleic acid amplification, and is capable of yielding an RNA amplification of a billion fold in 90 minutes. With respect to other amplification systems such as the PCR technique, the ability of NASBA to homogeneously and isothermally amplify RNA analytes extends its application range from viral diagnostics to the indication of biological activities such as gene expression and cell viability. NASBA technology is discussed, for example, in Nature volume 350 pages 91 and 92. Nucleic acid amplification in NASBA is accomplished by the concerted enzyme activities of AMV reverse transcriptase, Rnase H, and T7 RNA polymerase, together with a primer pair, resulting in the accumulation of mainly single-stranded RNA that can readily be used for detection by hybridization methods. The application of an internal RNA standard to NASBA results in a quantitative nucleic acid detection method with a dynamic range of four logs but which needed six amplification reactions per quantification. This method is improved dramatically by the application of multiple, distinguishable, internal RNA standards added in different amounts and by electrochemiluminesence (ECL) detection technology. This one-tube quantitative (Q) NASBA needs only one step of the amplification process per quantification and enables the addition of the internal standards to the clinical sample in a lysis buffer prior to the actual isolation of the nucleic acid. This approach has the advantage that the nucleic acid isolation efficiency has no influence on the outcome of the quantitation, which in contrast to methods in which the internal standards are mixed with the wild-type nucleic acid after its isolation from the clinical sample. Quantitative NASBA is discussed in Nucleic Acid Research (1998) volume 26, pages 2150-2155. Post-NASBA product detection, however, can still be a labour-intensive procedure, normally involving enzymatic bead-based detection and electrochemiluminescent (ECL) detection or fluorescent correlation spectrophotometry. However, as these methodologies are heterogeneous or they require some handling of sample or robotic devices that are currently not cost-effective they are relatively little used for high-throughput applications. A homogeneous procedure in which product detection is concurrent with target amplification by the generation of a target-specific signal would facilitate large-scale screening and full automation. Recently, a novel nucleic acid detection technology, based on probes (molecular beacons) that fluoresce only upon hybridization with their target, has been introduced.

Fluidics is the science of liquid flow in, for example, tubes. For microfabricated devices, flow of a fluid through the one or more sets of micro or nano sized reaction chambers is typically achieved using a pump such as a syringe, rotary pump or precharged vacuum or pressure source external to the device. Alternatively, a micro pump or vacuum chamber, or lamb wave pumping elements may be provided as part of the device itself. Other combinations of flow control elements including pumps, valves and precharged vacuum and pressure chambers may be used to control the flow of fluids through the reaction chambers. Other mechanisms for transporting fluids within the system include electro-osmotic flow.

International patent application publication no. WO 02/22265 relates to a microfabricated reaction chamber system, which may be used in a method of carrying out NASBA. International patent application no. WO 2003/060157 relates to a microfabricated reaction chamber system and a method of fluid transport. The system may also be used in a method of carrying out NASBA. International patent application no. WO 2003/809802 relates to a microfluidic device for nucleic acid fragmentation. The device may be used in or conjunction with a microfabricated reaction chamber system for carrying out NASBA.

US 2002045246 discloses a device for use with an ultrasonic transducer to lyse components of a fluid sample.

US 6544734 discloses a multilayered microfluidic DNA analysis system including a cell lysis chamber, a DNA separation chamber, a DNA amplification chamber, and a DNA detection system.

US 2003138941 discloses an apparatus comprising a substrate having at least one assay station, for detecting and diagnosing diseases and/or detecting amplified nucleic acid products and/or for pharmacogenetic determinations.

JOON-HO KIM ET AL: "A disposable DNA sample preparation microfluidic chip for nucleic acid probe assay" PROCEEDINGS OF THE IEEE 15TH. ANNUAL INTERNATIONAL CONFERENCE ON MICROELECTRO MECHANICAL SYSTEMS. MEMS 2002. LAS VEGAS, NV, JAN. 20 - 24, 2002, IEEE INTERNATIONAL MICRO ELECTRO MECHANICAL SYSTEMS CONFERENCE, NEW YORK, NY : IEEE, US, vol. CONF. 15, 2002, pages 133-136, XP010577613 ISBN: 0-7803-7185-2, discloses a microfluidic chip comprising a microfilter, a micromixer and a DNA purification chip.

WO 0062931 discloses a microfluidic device for the detection of a target analyte in a sample.

The present invention provides an integrated lab-on-a-chip diagnostic system for carrying out a sample preparation process on a fluid sample containing cells and/or particles, the system comprising the following components formed on a common substrate:
(a) an inlet for a fluid sample;
(b) a lysis unit for lysis of cells and/or particles contained in the fluid sample;
(c) a nucleic acid extraction unit for extraction of nucleic acids from the cells and/or particles contained in the fluid sample ;
(d) a reservoir containing a lysis fluid;
(e) a reservoir containing an eluent for removing nucleic acids collected in the nucleic acid extraction unit;
wherein the sample inlet is in fluid communication with the lysis unit, an optional valve being present to control the flow of fluid therebetween;
wherein the lysis unit is in fluid communication with the nucleic acid extraction unit, an optional valve being present to control the flow of fluid therebetween;
wherein the reservoir containing the lysis fluid is in fluid communication with the lysis unit, a valve being present to control the flow of fluid therebetween; and
wherein the reservoir containing the eluent is in fluid communication with the nucleic acid extraction unit, a valve being present to control the flow of fluid therebetween; and
wherein the system further comprises a single pump or syringe for actuation of all liquids.

The system can be used on millilitre sample volumes for routine diagnostics. The system relies on certain reagents being pre-loaded.

In the present invention nucleic acid extraction and concentration can be combined. Accordingly, the present invention provides an integrated lab-on-a-chip diagnostic system for carrying out a sample preparation process. The system may be used in or in conjunction with a microfabricated reaction chamber system for carrying out NASBA.

At least some of the components of the system are preferably microfabricated. The lysis unit, the nucleic acid extraction unit, the lysis fluid reservoir and the eluent reservoir are microfabricated and integrated, i.e. formed on a common substrate.

The reservoir containing the lysis fluid is in fluid communication with the inlet, a valve being present to control the flow of fluid therebetween.

The reservoir containing the eluent is in fluid communication with the inlet, a valve being present to control the flow of fluid therebetween.

The system according to the present invention will typically further comprise (g) a nucleic acid reaction unit, wherein the nucleic acid extraction unit is in fluid communication with the nucleic acid reaction unit, an optional valve being present to control the flow of fluid therebetween. Preferably, the nucleic acid reaction unit is microfabricated and preferably integrated with the other components. Any conventional reaction may be carried out in the reaction unit. Preferably, the reaction will enable detection of specific target sequence and/or quantitative analysis. The nucleic acid reaction unit will typically comprise a nucleic acid sequence amplification and detection unit, which enables detection of specific sequences by a nucleic acid amplification reaction. Examples include PCR and isothermal amplification techniques such as NASBA. The most preferred is real-time NASBA using molecular beacons. Accordingly, in a preferred aspect, the present invention provides an integrated lab-on-a-chip diagnostic system for carrying out a sample preparation, nucleic acid sequence amplification and detection process on a fluid sample containing cells and/or particles, more preferably real time NASBA. International patent application publication no. WO 02/22265 describes a microfabricated reaction chamber system for carrying out NASBA.

The system according to the present invention preferably involves concentration of, for example, infected epithelial cells, lysis and extraction of mRNA, and real-time amplification and detection.

The system may be used for the screening of cervical carcinoma, for example.

The system according to the present invention will typically further comprise (h) a waste unit, wherein the waste unit is in fluid communication with the lysis unit, an optional valve being present to control the flow of fluid therebetween. Preferably, the waste unit is microfabricated and preferably integrated with the other components.

The system will typically further comprise (i) a reservoir containing a washing solvent, which reservoir is in fluid communication with the nucleic acid extraction unit, an optional valve being present to control the flow of fluid therebetween. Preferably, the reservoir containing the washing solvent is microfabricated and preferably integrated with the other components. The washing solvent may be chosen from any suitable solvent, but preferably is one which can be readily evaporated, for example ethanol.

The system will typically further comprise (j) a reservoir containing a washing solvent, which reservoir is in fluid communication with the nucleic acid extraction unit, an optional valve being present to control the flow of fluid therebetween. Preferably, the reservoir containing the washing solvent is microfabricated and preferably integrated with the other components. The washing solvent may be chosen from any suitable solvent, but preferably is one which can be readily evaporated, for example isopropanol.

The reservoir containing the eluent is advantageously in fluid communication with the reservoir containing the first washing solvent (eg ethanol) and/or the reservoir second washing solvent (eg isopropanol).

More advantageously, the eluent, the first washing solvent (eg ethanol) and/or the second washing solvent (eg isopropanol) are contained in a common reservoir. This may be achieved by separating the eluent, the first washing solvent and/or the second washing solvent from one another in the common reservoir by the use of a fluid such as, for example, air. Other "separating" fluids (liquids or gases) can be used, however, as long as they are immiscible or at least substantially immiscible with the eluent, the first washing solvent and/or the second washing solvent.

In a preferred embodiment, the eluent, the ethanol and/or the isopropanol are contained in a conduit or channel which is in fluid communication with the inlet and the lysis unit. The eluent, the ethanol and/or the isopropanol being separated by fluid gaps such as air gaps, for example.

The system will typically further comprise (k) means for introducing a fluid sample and/or air into the inlet. Said mean preferably comprising a pump or a syringe. Alternatively, such means may comprises one or more variable volume chambers in communication with the inlet port, wherein altering the volume of the variable volume chamber(s) effects and/or restricts flow of a fluid sample into and/or out of the inlet. The variable volume chamber typically comprises a flexible membrane overlying a hollow recess in the underlying substrate. International patent application no. WO 2003/060157 describes a preferred fluid transport system.

The system is advantageously driven by a single pump or syringe.

The lysis unit may have any suitable shape and configuration but will typically be in the form of a channel or chamber. The lysis unit is preferably for lysis of eukaryotic and prokaryotic cells and particles contained in the fluid sample.

If desired, the system may further comprise a filtration unit, which unit is in fluid communication with the lysis unit. The filtration unit may comprise, for example, a cross-flow filter or a hollow filter. Alternatively, the lysis unit may itself further comprise means to filter the fluid sample. Said mean may comprise, for example, a cross-flow filter or a hollow filter, which may be integrated with the lysis unit.

If desired, the system may further comprise a fragmentation unit, which unit is in fluid communication with the lysis unit. Alternatively, the lysis unit may itself further comprise means to fragment the fluid sample. Random fragmentation of DNA or RNA is often necessary as a sample pre-treatment step. Fragmentation may be achieved biochemically using restriction enzymes, or through application of a physical force to break the molecules (see, for example, P. N. Hengen, Trends in Biochem. Sci. , vol. 22, pp. 273- 274, 1997 and P. F. Davison, Proc. Nat. Acad. Sci. USA , vol. 45, pp. 1560- 1568, 1959). DNA fragmentation by shearing usually involves passing the sample through a short constriction. In a preferred embodiment, DNA and/or RNA breaks under mechanical force when pumped through a narrow orifice, due to rapid stretching of the molecule. A pressure-driven flow can lead to a shear force, which leads to fragmentation of the nucleic acids. International patent application no. PCT/GB03/004768 describes a microfluidic device for nucleic acid fragmentation.

The lysis unit may itself further comprise means to filter the fluid sample and means to fragment the fluid sample.

The system may further comprises means for heating the contents of the lysis unit and/or the nucleic acid extraction unit. Said mean may comprise, for example, one or more Peltier elements located in or adjacent the lysis unit and/or the nucleic acid extraction unit.

The nucleic acid extraction unit may have any suitable shape and configuration but will typically be in the form of a channel or chamber. The nucleic acid extraction unit is preferably for extraction of eukaryotic and prokaryotic cells and particles contained in the fluid sample.

The nucleic acid extraction unit may be at least partially filled with silica beads or particles. One or more sets of electrodes may be provided adjacent the silica beads or particles for collecting and/or pre-concentrating the eluted nucleic acids. The one or more sets of electrodes may comprise platinum electrodes, for example. Means may therefore be provided for applying a potential difference across the electrodes. The extraction cell is preferably formed from or comprises poly(dimethylsiloxane) (PDMS). The unit will typically comprise a substrate and an overlying cover, the extraction unit being defined by a recess in a surface of the substrate and the adjacent surface of the cover. The substrate is preferably formed from silicon poly(dimethylsiloxane) (PDMS). The NA binds to silica surfaces in the presence of chaotropic agents.

The integration of electrodes (eg platinum electrodes) may advantageously be used to reversibly collect and pre-concentrate the eluted NA on-chip. Thus, the present invention enables combined nucleic acid extraction and enrichment to be achieved.

In a preferred embodiment, the nucleic acid extraction unit comprises a silica bead-packed poly(dimethylsiloxane) (PDMS) channel.

The system or at least a master version thereof will typically be formed from or comprise a semiconductor material, although dielectric (eg glass, fused silica, quartz, polymeric materials and ceramic materials) and/or metallic materials may also be used. Examples of semiconductor materials include one or more of: Group IV elements (i.e. silicon and germanium); Group III-V compounds (eg gallium arsenide, gallium phosphide, gallium antimonide, indium phosphide, indium arsenide, aluminium arsenide and aluminium antimonide); Group II-VI compounds (eg cadmium sulphide, cadmium selenide, zinc sulphide, zinc selenide); and Group IV-VI compounds (eg lead sulphide, lead selenide, lead telluride, tin telluride). Silicon and gallium arsenide are preferred semiconductor materials. The system may be fabricated using conventional processes associated traditionally with batch production of semiconductor microelectronic devices, and in recent years, the production of semiconductor micromechanical devices. Such microfabrication technologies include, for example, epitaxial growth (eg vapour phase, liquid phase, molecular beam, metal organic chemical vapour deposition), lithography (eg photo-, electron beam-, x-ray, ion beam-), etching (eg chemical, gas phase, plasma), electrodeposition, sputtering, diffusion doping, ion implantation and micromachining. Non-crystalline materials such as glass and polymeric materials may also be used.

Examples of polymeric materials include PMMA (Polymethyl methylacrylate), COC (Cyclo olefin copolymer), polyethylene, polypropylene, PL (Polylactide), PBT (Polybutylene terephthalate) and PSU (Polysulfone), including blends of two or more thereof. The preferred polymer is PDMS or COC.

The device/system will typically be integrally formed. The device/system may be microfabricated on a common substrate material, for example a semiconductor material as herein described, although a dielectric substrate material such as, for example, glass or a ceramic material could be used. The common substrate material is, however, preferably a plastic or polymeric material and suitable examples are given above. The system may preferably be formed by replication of, for example, a silicon master.

The advantages of using plastics instead of silicon-glass for miniaturized structures are many, at least for biological applications. One of the greatest benefits is the reduction in cost for mass production using methods like microinjection moulding, hot embossing and casting. A factor of a 100 or more is not unlikely for complex structures. The possibility to replicate structures for multilayered mould inserts gives a great flexibility of design freedom. Interconnection between the micro and macro world are in many cases easier because one got the option to combine standard parts normally used. Different approaches can be used for assembly techniques, like e.g. US-welding with support of microstructures, laser welding, gluing and lamination. Other features that are profitable is surface modification. For miniaturized structures addressed for biological analysis, it is important that the surface is biocompatible. By utilizing plasma treatment and plasma polymerization a flexibility and variation of assortment can be adapted into the coating. Chemical resistance against acids and bases are much better for plastics than for silicon substrates that are easily etched away. Most detection methods within the biotechnological field involves optical measurements. The transparency of plastic is therefore a major feature compared to silicon that are not transparent. Polymer microfluidic technology is now an established yet growing field within the Lab-on-a-chip market.

The microfabricated system as herein described is also intended to encompass nanofabricated devices.

For a silicon or semiconductor master, it is possible to define by, for example, etching or micromachining, one or more of variable volume chambers, microfluidic channels, reaction chambers and fluid interconnects in the silicon substrate with accurate microscale dimensions. A plastic replica may then be made of the silicon master. In this manner, a plastic substrate with an etched or machined microstructure may be bonded by any suitable means (for example using an adhesive or by heating) to a cover.

The optional valves used in the system may take any convenient form. For example, the valves may simply regulate flow along a conduit or channel connecting two units. A piston-like member may be provided which can be raised or lowered in a hole in a conduit or channel by the action of a pin device.

Use of the system involves the following possible steps, by way of example.

### Alternative 1

(i) Sample collection and lysis
(ii) Extraction of mRNA (manual or automatic procedure)
(iii) Real-time amplification and detection (preferably multiplex)

### Alternative 2

(iv) A fragmentation unit may include both sample lysis and sample preparation
(v) Real-time amplification (NASBA) and detection (preferably multiplex).

A method for the manufacture of an integrated lab-on-a-chip diagnostic system is herein described, which method comprises:
A. providing a substrate having an inlet recess, a lysis unit recess, a nucleic acid extraction unit recess, a lysis fluid reservoir recess and an eluent reservoir recess in a surface thereof;
B. providing a cover ; and
C. bonding the cover to the substrate to create the (a) inlet, (b) the lysis unit, (c) the nucleic acid extraction unit, (d) the lysis fluid reservoir and (e) the eluent reservoir, each being defined by the respective recess in said surface of the substrate and the adjacent surface of the cover.

The term recess as used herein is also intended to cover a variety of features including, for example, grooves, slots, holes, trenches and channels, including portions thereof.

The method may further comprise the step of introducing lysis fluid into the lysis fluid reservoir either before or after bonding the cover to the substrate.

The method may further comprise the step of introducing eluent into the eluent reservoir either before or after bonding the cover to the substrate.

The method may further comprise the step of introducing ethanol into the eluent reservoir either before or after bonding the cover to the substrate.

The method may further comprise the step of introducing isopropanol into the eluent reservoir either before or after bonding the cover to the substrate.

The eluent, and/or the ethanol and/or the isopropanol are preferably separated from one another by a fluid, preferably air, although any immisible fluid (liquid or gas) may be used.

The method may comprise:
introducing eluent into the eluent reservoir after bonding the cover to the substrate ;
introducing a first volume of air into the eluent reservoir ;
introducing ethanol into the eluent reservoir, whereby the ethanol is separated from the eluent by said first volume of air ;
introducing a second volume of air into the eluent reservoir ;
introducing isopropanol into the eluent reservoir, whereby the isopropanol is separated from the ethanol by said second volume of air.

The substrate may be formed from silicon, for example, and the overlying cover from glass, for example. In this case, the glass cover is preferably anodically bonded to the silicon substrate, optionally through an intermediate silicon oxide layer formed on the surface of the substrate. The recesses in the silicon may be formed using reactive-ion etching. Other materials such as polymeric materials may also be used for the substrate and/or cover. Such materials may be fabriacted using, for example, a silicon replica. Alternatively, the device may be fabricated by structuring of mould inserts by milling and electro-discharge machining (EDM), followed by injection moulding of the chip parts, followed by mechanical post-processing of the polymer parts, for example drilling, milling, debarring. This may subsequently be followed by insertion of the filter, solvent bonding, and mounting of fluidic connections.

Examples of polymeric materials include PMMA (Polymethyl methylacrylate), COC (Cyclo olefin copolymer), polyethylene, polypropylene, PL (Polylactide), PBT (Polybutylene terephthalate) and PSU (Polysulfone), including blends of two or more thereof. COC is preferred.

Preferably, and in particular if optical observations of the contents of the cell are required, the overlying cover is made of an optically transparent substance or material, such as glass, Pyrex or COC.

Combinations of a microfabricated component with one or more other elements such as a glass plate or a complementary microfabricated element are frequently used and intended to fall within the scope of the term microfabricated used herein.

Part or all of the substrate base may be provided with a coating of thickness typically up to 1 µm, preferably less than 0.5 µm. The coating is preferably formed from one or more of the group comprising polyethylene glycol (PEG), Bovine Serum Albumin (BSA), tweens and dextrans. Preferred dextrans are those having a molecular weight of 9,000 to 200,000, especially preferably having a molecular weight of 20,000 to 100,000, particularly 25,000 to 75,000, for example 35,000 to 65,000). Tweens (or polyoxyethylene sorbitans) may be any available from the Sigma Aldrich Company. PEGs are preferred as the coating means, either singly or in combination. By PEG is embraced pure polyethylene glycol, i.e. a formula HO-(CH₂CH₂O)ₙ-H wherein n is an integer whereby to afford a PEG having molecular weight of from typically 200 - 10,000, especially PEG 1,000 to 5,000; or chemically modified PEG wherein one or more ethylene glycol oligomers are connected by way of homobifunctional groups such as, for example, phosphate moieties or aromatic spacers. Particularly preferred are polyethylene glycols known as FK108 (a polyethylene glycol chain connected to another through a phosphate); and the PEG sold by the Sigma Aldrich Company as product P2263. The above coatings applied to the surfaces of the cell/chamber, inlets, outlets, and/or channels can improve fluid flow through the system. In particular, it has been found that the sample is less likely to adhere or stick to such surfaces. PEG coatings are preferred.

For a silicon or semiconductor master, it is possible to define by, for example, etching or micromachining, one or more of variable volume chambers, microfluidic channels, reaction chambers and fluid interconnects in the silicon substrate with accurate microscale dimensions (deep reactive-ion etching (DRIE) is a preferred technique). A plastic replica may then be made of the silicon master. In this manner, a plastic substrate with an etched or machined microstructure may be bonded by any suitable means (for example using an adhesive or by heating) to a cover thereby forming the enclosed fragmentation cell(s), inlet(s), outlet(s) and connecting channel(s).

The device comprises a substrate with the desired microstructure formed in its upper surface. The substrate may be silicon, for example, or a plastic substrate formed by replication of a silicon master. The substrate is bonded at its upper surface to a cover, thereby defining a series of units/cells, inlets, outlets, and/or channels. The cover may be formed from plastic or glass, for example. The cover is preferably transparent and this allows observation of the fluid. In general, the device is preferably fabricated by deep reactive-ion etching (DRIE) of silicon for high aspect ratio constrictions, followed by anodic bonding of a glass cover. Alternatively, the device may be fabricated by structuring of mould inserts by milling and electro-discharge machining (EDM), followed by injection moulding of the chip parts, followed by mechanical post-processing of the polymer parts, for example drilling, milling, debarring. This may subsequently be followed by insertion of the filter, solvent bonding, and mounting of fluidic connections.

The nucleic acid sample may be or be derived from, for example, a biological fluid, a dairy product, an environmental fluids and/or drinking water. Examples include blood, serum, saliva, urine, milk, drinking water, marine water and pond water. For many complicated biological samples such as, for example, blood and milk, it will be appreciated that before one can isolate and purify DNA and/or RNA from bacterial cells and virus particles in a sample, it is first necessary to separate the virus particles and bacterial cells from the other particles in sample. It will also be appreciated that it may be necessary to perform additional sample preparation steps in order to concentrate the bacterial cells and virus particles, i.e. to reduce the volume of starting material, before proceeding to break down the bacterial cell wall or virus protein coating and isolate nucleic acids. This is important when the starting material consists of a large volume, for example an aqueous solution containing relatively few bacterial cells or virus particles. This type of starting material is commonly encountered in environmental testing applications such as the routine monitoring of bacterial contamination in drinking water.

The system is preferably designed to cater for a sample volume of 10-100 ml.

The present invention also provides an apparatus for the analysis of biological and/or environmental samples, the apparatus comprising a system as herein described. The apparatus may be a disposable apparatus.

The present invention also provides an assay kit for the analysis of biological and/or environmental samples, the kit comprising a system as herein described and means for contacting the sample with the system. The assay kit may be a disposable kit.

The present invention will now be described, by way of example, with reference to the accompanying drawings, of which:
Figure 1 is a schematic illustration of a sandwich layout used for integration of a flat membrane into a disposable polymer chip device for use in the present invention.
Figure 2 is a schematic illustration of a valve design for use with the system according to the present invention.
Figures 3a-d are schematic illustrations of a valve design for use with the system according to the present invention.
Figure 4 is a schematic illustration a possible layout of a bead chamber according to the present invention.
Figures 5 is a schematic illustration of a system design according to the present invention showing filling with lysis buffer (Figure 5a) and extraction fluids (Figure 5b).
Figure 6 is a schematic illustration of a chip layout according to a preferred embodiment of the present invention.
Figure 7 is a schematic illustration of a system design according to another preferred embodiment of the present invention.
Figure 8 relates to the Examples.
Figure 9 relates to the Examples.

A plastic chip design according to the present invention preferably incorporates supply channels, reaction chambers and microfluidic actuation systems and is preferably processed by injection moulding of cycloolefin copolymer (COC). The mould insert for, for example, a 12-channel chip may be manufactured using high precision milling. The detection volume is typically approximately 80 nL (400 × 2000 × 100 µm). The plastic chip is preferably first oxygen plasma activated before being coated with a 5% polyethylene glycol (PEG) solution (Sigma Chemical Co, St. Louis, MO). After coating, the chip may be sealed with an approximately 75 µm COC membrane via solvent welding using, for example, bicyclohexcyl. A thin gold layer (approx 25 nm) is preferably deposited on the backside of the chip to prevent background fluorescence from the thermal pad on top of the Peltier element.

If required, Peltier elements may be integrated into the sample holder providing thermal control for the plastic chips. Aluminium blocks may be put on top of the Peltier elements to secure an even distribution of heat for the chips. A thermal pad is preferably mounted on the aluminium blocks to establish thermal contact between the chips and the heating source. A thermocouple will typically be placed on the sample holder measuring the air temperature and having a feedback circuit to the Peltier elements. The temperature regulation can be controlled externally on a laptop.

As previously described, NASBA is an isothermal (approximately 41°C) amplification method specifically designed for amplifying any single-stranded RNA sequence. The NASBA reaction can be applied to a wide range of applications such as detection of the presence of specific viral RNAs, RNAs of other infectious or pathogenic agents or certain cellular RNAs. Simultaneous activity of the three enzymes, AMV Reverse Transcriptase, RNase H and T7 RNA polymerase makes the core technology in the amplification reaction. Two oligonucleotide primers determine the specificity of the reaction and fluorescent molecular beacon probes that are specific for the target RNA. In approximately 90 minutes the nucleic acid sequence of interest can be amplified to > 10⁹ copies. The optical detection unit is preferably designed to excite the fluorophores in the reaction chambers at approximately 494 nm and detect the emitted fluorescent light at approximately 525 nm. The excitation light may be filtered using a bandwidth filter (465 nm - 500 nm) before the light is collimated through a lens. The same Fresnel lens may be used for focusing the illumination and collection of the fluorescence light. Another lens may be used to focus the fluorescent light onto the detector surface (eg a photomultiplier-tube). The data collection and preparation of the detected signal may be processed on a laptop using MATLAB 6.0.088 Release 12 (The MathWorks Inc., Natick, MA).

Efficient sample pre-treatment is an important factor in the context of micro-technological analysis systems. In particular, concentration devices are needed in order to enable detection of low numbers of specific particles, as e.g. cells bacteria or viruses, present in biological samples. A variety of concentration methods are known in the art including, for example, filtration techniques such as dead-end filtration and cross-flow filtration using different kinds of filtration media (micro-structured channels, porous hollow fibres or membranes), gravity settlers, centrifuges, acoustic cell filters, optical traps, dielectrophoresis (DEP), electrophoresis, flow cytometry and adsorption based methods.

A preferred method of concentration involves dead-end filtration. This is a relatively simple and cheap method, which can readily be integrated into a disposable polymer chip. Furthermore, the use of flat membranes assures a high flexibility concerning the field of application, since a variety of membranes are available and surface treatments such as, for example, PEG or Tween20 coating can easily be performed.

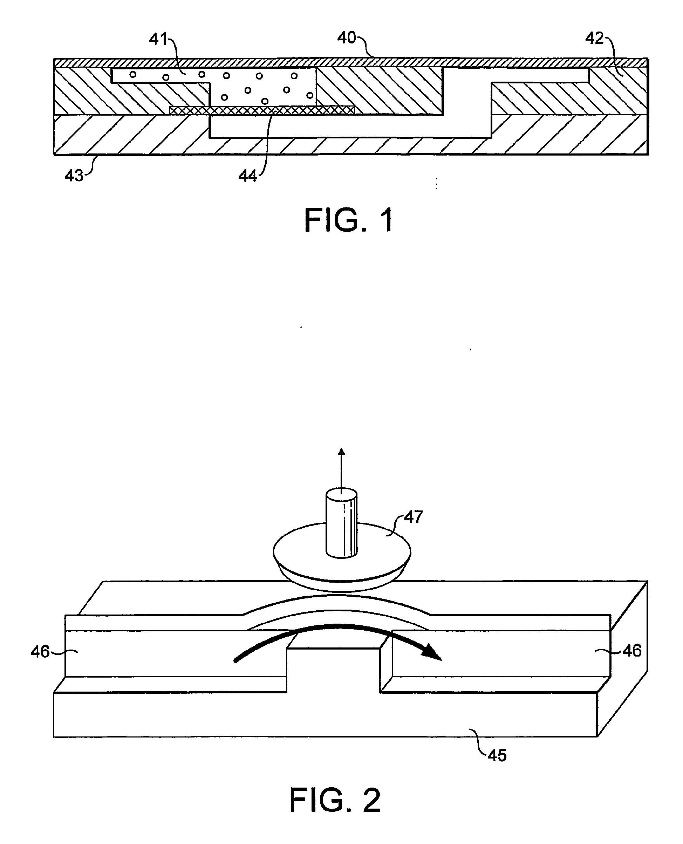

The integration of a flat membrane into a polymer disposable chip may be achieved using a sandwich set-up as shown schematically in Figure 1. The chip comprises a cover membrane 40, a fluid channel 41, and a filter membrane 44. The top and bottom of the chip are shown as 42 and 43 respectively.

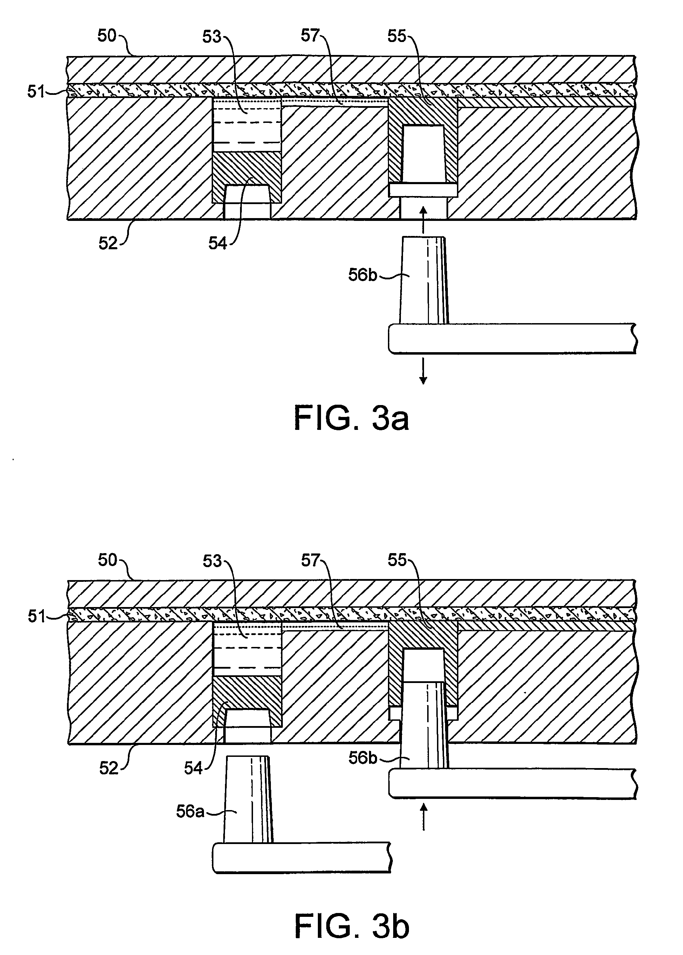

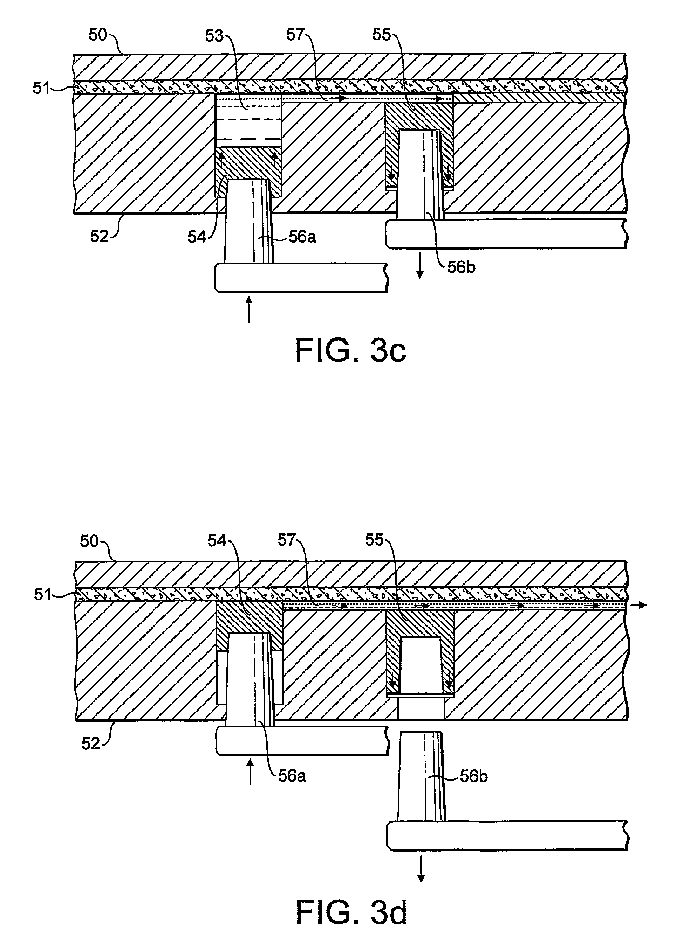

Valves are integrated into the device in order to enable a flow control on-chip according to claim 1. Suitable valve designs are shown in Figures 2 and 3. With regard to Figure 2, pre-shaped membranes or flat membranes may be used. The chip 45 comprises a fluid channel 46 and a pre-shaped membrane 47. The vertical arrow indicates the open position.

With regard to Figures 3 a-d, there is shown a chip having a body, which comprises a top body portion 50, a main body portion 52, and a membrane 51 interposed therebetween. A microfluidic channel 57 is provided adjacent the membrane 51. A piston 54 and a valve 55 are provided in suitable recesses in the main body portion 52. Fluid/liquid is present in a volume 53 above the piston 54 (see Figure 3a). The valve 55 is mounted with interference fit in the upper position (see Figure 3a). In this position it seals the microfluidic channel 57 so that no fluid may pass. A conic pin 56b may be used to lower the valve 55 to the open position (see Figures 3b, 3c and 3d). In particular, when the pin 56b is pushed upwards it is secured, by a friction fit, in a corresponding recess in the valve 55. Similarly, in relation to piston 54, when the conic pin 56a is pushed upwards it is secured, by a friction fit, in a corresponding recess in the piston 54. In order to transport the liquid from the volume 53, pins 56a and 56b are pushed into the corresponding recesses in the piston 54 and the valve 55 respectively and the liquid is pushed out of the volume 53 (see Figures 3c and 3d). When the chip has been used the conic pins are 56a and 56b are withdrawn from the piston 54 and the valve 55 respectively.

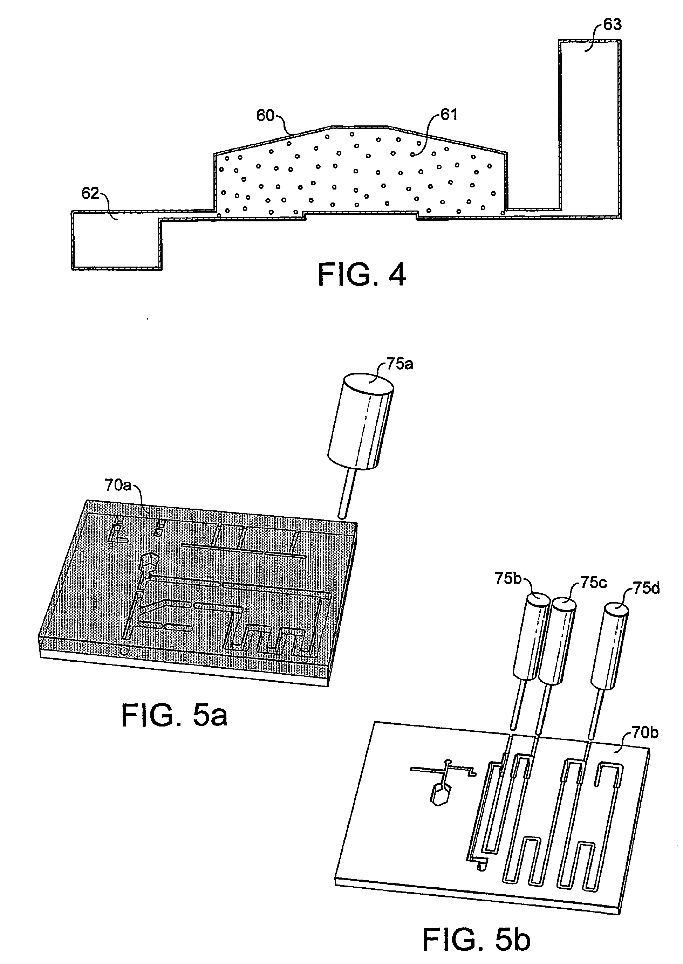

The inventors have found that silica beads are well suited for RNA extraction and purification. Typically 0.3-0.4 mg of beads with diameters of 15 µm to 35 µm can be used for extraction, but is also possible to use larger silica beads (up to approximately 200 µm diameter). A possible layout of a bead chamber is shown in Figure 4. The bead chamber 60 is loaded prior to chip-to-chip bonding with pre-wetted silica beads 61. After bonding, the bead package is retained by the 100 µm bottlenecks. The shape of the bead chamber and the arrangement of the fluidic connections 62 (inlet) and 63 (outlet) ensure that the applied liquid passes the silica beads 61, even if the bead chamber 60 is not filled completely. The volume of the bead chamber 60 is about 6.5 µL and is suitable for extraction from a sample of typically 10 to 50 µL.

Four liquids are preferably used throughout the pre-treatment process: lysis buffer (typically approx 100 µL), isopropanol (typically approx 40 µL), ethanol (typically approx 40 µL), and elution buffer (typically approx 5-20, µL). The latter three are needed for extraction. The inventors have found that it is advantageous to store the lysis buffer in a channel (typically a meandering channel) on the top chip 70a (see Figure 5a) and storage of the extraction liquids in two W-shaped and one U-shaped reservoirs on the bottom part 70b (see Figure 5b).

All of the storage reservoirs may simply be filled by means of small (0.5 mm x 0.5 mm) side channels, indicated in the Figure 5 by the needle positions of the outlined syringes 75a-d. After filling, the side channels can be sealed using any appropriate means, such as with liquid glue or tape.

Advantageously, in order to allow for a relatively simple handling system, a single (syringe) pump for actuation of all liquids is used.

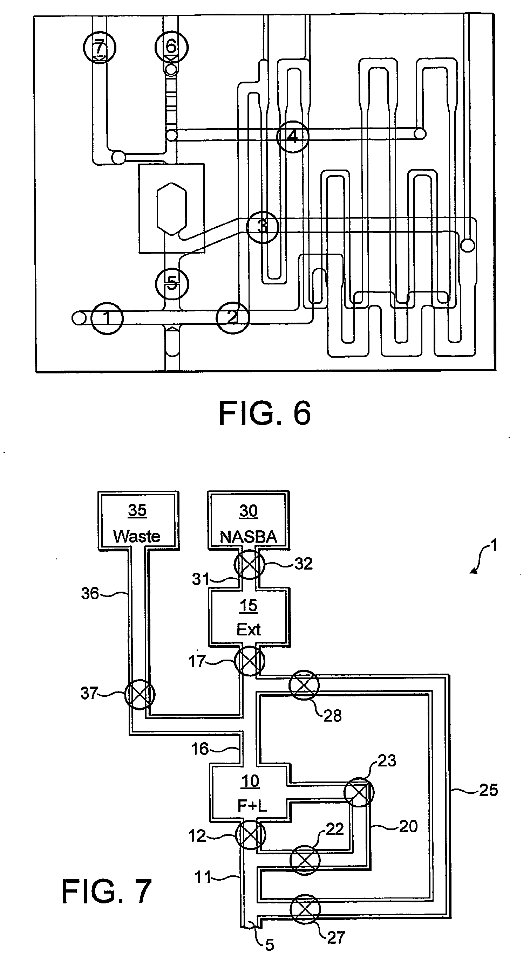

A chip layout according to a preferred embodiment of the present invention is shown in Figure 6.

The four liquid reservoirs (lysis buffer, isopropanol, ethanol, and elution buffer) are sequentially filled using conventional syringes (needle diameter 0.4 mm), and the filling channels are sealed.

First, the cell suspension is applied to the filtration unit by means of a syringe pump. Besides particulate suspension the syringe is loaded with about 200 µL to 300 µL of air, which is used for actuation of the on-chip liquids (Depending on the application it will be appreciated that other immiscible liquids may be used).

Second, air is pumped into the lysis buffer reservoir and the displaced buffer is applied to the cells being kept on the filter. The cell lysate is pushed through the filter and is directed to the beads chamber. Due to the additional filtering step the probability of clogging in the beads chamber is reduced.

Third, the actuation pump (syringe) is connected to the extraction liquid reservoir while the connections to the filter chamber and the lysis buffer reservoir are closed. The extraction liquids are stored in a single reservoir separated by air plugs. When pressure is applied to one side of the reservoir, the liquids are displaced in parallel and are sequentially guided through the beads chamber.

The operation protocol including the valve operations is summarized below with reference also to Figure 6. Valves not listed are in a closed state, whereas the listed valves are opened for the corresponding operation.

### Filtration

| | |
|---|---|
| Valves 5, 7: | Cell suspension in, filtrate -> Left Outlet |

### Lysis

| | |
|---|---|
| Valves 2, 3, 7: | Air in, displaced fluid -> Left Outlet |
| Valves 2, 3, 6: | Air in, lysate -> bead package, Right Outlet |

### Purification

| | |
|---|---|
| Valves 1, 4, 6: | Air in, isopropanol -> bead package Air in, ethanol -> bead package Air in, elution buffer -> bead package |

Turning now to Figure 7, which shows another preferred embodiment of the present invention. The foregoing description is also applicable to this embodiment. The system 1 comprises an inlet 5 for a fluid sample, a lysis/filtration unit 10, a nucleic acid extraction unit 15, a channel 20 containing lysis fluid, a channel 25 containing eluent, ethanol and isopropanol, a nucleic acid sequence amplification and detection unit 30, and a waste unit 35.

A channel 11 connects the sample inlet 5 to the lysis/filtration unit 10. A valve 12 is provided to control the flow of fluid therebetween.

A channel 16 connects the lysis/filtration unit 10 to the nucleic acid extraction unit 15. A valve 17 is provided to control the flow of fluid therebetween.

The channel 20 containing the lysis fluid is connected to the lysis/filtration unit 10 and the sample inlet 5. Valve 22s and 23 are provided to control the flow of fluid.

The channel 25 containing the eluent, ethanol and isopropanol is connected to the nucleic acid extraction unit 15 and the sample inlet 5. Valves 27 and 28 are provided to control the flow of fluid.

A channel 31 connects the nucleic acid extraction unit 15 to the nucleic acid sequence amplification and detection unit 30. A valve 32 is provided to control the flow of fluid therebetween.

A channel 36 connects the lysis/filtration unit 10 to the waste unit 35. A valve 37 is provided to control the flow of fluid therebetween.

The channel 25 contains the eluent and washing solvents such as ethanol and isopropanol. The eluent and washing solvents are preloaded into the channel using an air gap to separate the liquids from one another.

An example of a suitable lysis buffer fluid is 100 mM Tris/HCl, 8 M GuSCN (pH 6.4).

An example of a suitable elution solution is 10 mM Tris/HCl, 1 mM EDTA Na₂ (pH 8) + 1 mM YOYO-1.

Nucleic acid quantification may be achieved using a fluorescence microscope and a pixel-intensity analysis program (Lispix).

The nucleic acid extraction unit contains silica beads, for example 0.3 mg of 15-30 µm size silica beads. Platinum elctrodes are also provided (not shown) just below the packed bed for electrokinetic collection of the negatively charged, eluting nucleic acids.

The operation protocol is summarized below.

### Filtration

All valves are closed except for valves 12 and 37. A syringe containing a fluid sample (which contains the cells to be analysed) is connected to the sample inlet 5 and the sample is injected under pressure into the filtration/lysis unit 10. In this way cells are retained in the unit 10 and the remaining protion of the fluid is then passed to the waste unit 35.

### Lysis

All valves are closed except for valves 22, 23 and 37. In a first step (optional), air contained in the syringe is injected into the sample inlet 5. This causes the lysis fluid contained in channel 20 to move towards the filtration/lysis unit 10. Before the lysis fluid enters the filtration/lysis unit 10, however, the air ahead of the lysis fluid, i.e. the air in the region of the channel 20 between the valve 23 and the unit 10, causes any remaining fluid in the unit 10 to be displaced and to flow to the waste unit 35. Next, in a second step, valve 37 is closed and valve 17 is opened. As air contained in the syringe continues to be injected into the sample inlet 5, the lysis fluid contained in the channel 20 flows under pressure into the filtration/lysis unit 10. As a consequence, the retained cells therein are lysed and the lysate flows to the nucleic acid extraction unit 15.

### Purification/Extraction

All valves are closed except for valves 27, 28 and 32. In a first step, air contained in the syringe is injected into the sampele inlet 5. This causes the fluids (isopropanol, air gap, ethanol, air gap, elution buffer) contained in channel 25 to move as a column of fluid towards the nucleic acid extraction unit 15. This process is halted once all of the isopropanol (i.e. the first portion of the column of fluid) has been passed into the nucleic acid extraction unit 15. After a short period of time (together with optional heating of the contents of unit 15), the process is continued and the air gap between the isopropanol and the ethanol displaces the isopropanol. The isopropanol evaporates and/or goes to waste. The ethanol then flows under pressure into the nucleic acid extraction unit 15. The process is once again halted once all the ethonal has passed into the unit 15. After a short period of time (together with optional heating of the contents of unit 15), the process is continued and the air gap between the ethanol and the elution buffer displaces the ethanol. The ethanol evaporates and/or goes to waste. The elution buffer then flows under pressure into the nucleic acid extraction unit 15 and elutes the nucleic acids released from the surface of the silica beads. The eluted nucleic acids then pass to the nucleic acid sequence amplification and detection unit 30.

The present invention provides an apparatus for nucleic acid (NA) extraction and analysis. Extraction from biological samples, such as human cell lysates, has been successful, with collection of the NA in the first 15 mL of eluate.

Real-time Nucleic acid sequence-based amplification (NASBA) has been measured in cycloolefin copolymer (COC) plastic microchips with incorporated supply channels and parallel reaction chambers. Successful detection of an artificial Human Papillomavirus (HPV) 16 sequence, a SiHa cell line with incorporated HPV 16 and patient samples tested positive for HPV 16 have been performed. The sample materials applied to the chip were divided into eleven parallel reaction chambers where it was simultaneously detected in a detection volume of 80 nL.

The following Examples are not in accordance with the present invention. The Examples are, however, provided to show a possible application of the invention.

### Examples

### Sample material

The cervical carcinoma cell lines SiHa (squamous cell carcinoma) were obtained from the American Type Culture Collection (USA). SiHa cell-line was maintained in Dulbecco's modified Eagles medium (DMEM), supplemented with 10% fetal bovine serum (FBS), 2 mM L-glutamine and 25 µg/ml gentamicin. The cells were incubated at 37°C in a 5% CO₂ atmosphere. The cells were trypsinated, counted in Bürkers chamber, and lysed in NASBA lysis buffer (bioMérieux, the Netherlands, containing 5 M guanidine thiocyanate). The nucleic acids were isolated and extracted using the Boom's method (Boom, R., Sol, J.A., Salimans, M. M. M., Jansen, C. L., Wertheimvandillen P. M. E., Vandernoordaa, J. J. of Clinical Microbiol., 1990,28,(3), 495-503.) on a NucliSense Extractor. SiHa cells contain 1-2 copies of integrated HPV 16 DNA per cell (Syrjanen, S., Partanen, P., Mantyjarvi, R., and Syrjanen, K. J virol Methods, 1988, 19, 225-238). A ten-fold serial dilutions of the SiHa cellline extract were tested. In addition, artificial HPV type 16 sequences, from the HPV Proofer kit (NorChip AS, Norway) was used as target. A dilution series were tested to define the detection limit of the system.

### NASBA

The reagents in the PreTect® HPV-Proofer kit were mixed according to the manufacturers specifications (NorChip AS, Norway). All primers and probes were available in the kit. Additionally, BSA was added to the mixture to a final concentration of 0.05 % as a dynamic coating. Reagent solution (26 µL) from the kit and 13 µL of sample material (SiHa cell-line samples and HPV type 16 sequence samples from the kit) were mixed and heated to 65°C for 2 minutes. The mixture was subsequently cooled to 41°C for 2 minutes after which the enzymes (13 µL) were added. One actuation chamber on each reaction channel was cut open before adding the mixture into the polymer microchip. Each reaction channel in the chip was filled with the mixture due to capillary forces. The remaining mixture was drawn into the waste chamber at the end at the supply channel. The chip holder was then moved under the optics, where one after the other channel was measured. Measurements were taken every 30 seconds. Only a 2 x 2 mm² area were illuminated by the LED, this area corresponded to a detection area of 80 nL. The ten-fold serial dilutions of both HPV 16 sequences and SiHa cell-lines were also tested with conventional equipment for comparison with microchip detection. All experiments were run for 2.5 hours.

### Calculation

All the results were calculated using PreTect Data Analyzer (PDA) (NorChip AS). The microchip was designed with 12 reaction chambers, but the two reaction channels on each side were removed in the calculations due to systematic error of the measurements. The calculations were based on polynomial regression algorithms. The ratio was defined as the difference in fluorescence level at the end of the reaction and the fluorescence level at the start of the reaction. All samples with a ratio of 1.7 or greater were defined to be positive. Time-to-positivity or the starting point were set to be where the curve started to increase exponentially. The average slop were calculated using the values of 10% increase in fluorescent level and the value of an 80% increase in fluorescent level from the starting point. The detection limit for the polymer microchips was set to be the last concentration tested where all the 10 reaction channels were positive.

### Results

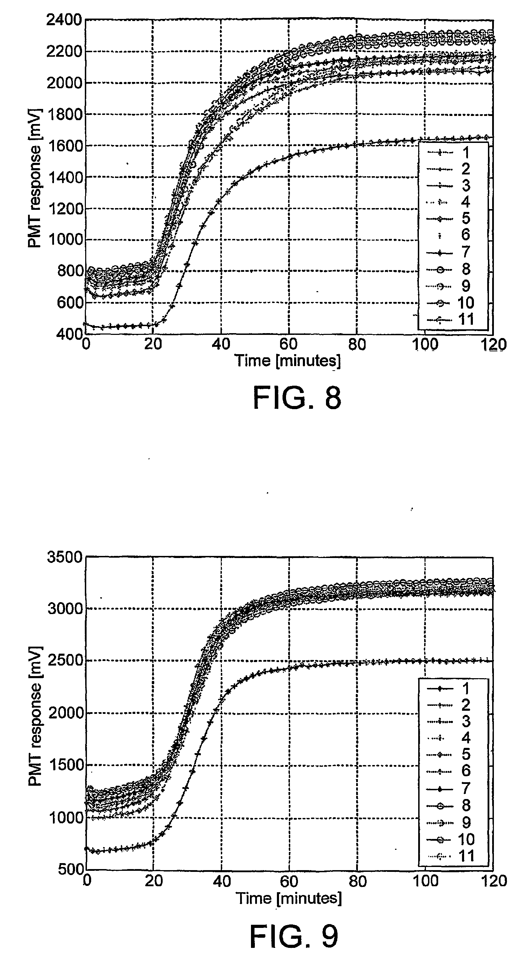

Identification of HPV 16 virus utilizing real-time NASBA was successfully performed in polymer microchips with a detection volume of 80 nL. Figures 8 and 9 illustrate the result from one experiment performed on SiHa cell-lines and HPV 16 oligo sequences, respectively. The Figures show graphs that clearly are positive and have the same curvature as samples performed using regular 20 µL volumes and conventional readers (not shown). Table 1 shows the results of a dilution series of artificial HPV16 sequences and SiHa cell-lines obtained using the polymer microchips. To characterize the amplification reactions, several different parameters were evaluated: the fluorescence ratio, time-to-positivity, the average slop of the linear part of the curve, the number of positive amplifications and the number of polymer microchips tested. The values in the table show the average value and the standard deviation of the positive samples that were tested. For both HPV 16 sequences and SiHa cell lines tested on the microchips, the ratio was more or less constant. In comparison with conventional testing (Table 2) of the same sample material, showed that the ratio were decreasing for lower concentrations. The other parameters on the other hand correspond very well for both the microchips and the conventional methods. Time-to-positivity increased with lower concentrations. While the average slop values decreased with lower concentrations. Ten-fold serial dilutions from 100 aM to 100 nM were tested for artificial HPV 16 sequences, while SiHa cell-line were tested for 0.02 cells/µl to 2000 cells/µl. The custom-made optical detection system had a detection limit of 1 pM and 20 cells/µl for artificial HPV 16 sequences and SiHa cell-line material, respectively. These were the same detection limits obtained for the conventional Biotek readers. It was possible to detect lower concentrations on both systems but the results were not consistent. The results also illustrate that when the sample concentration of input target were decreasing, the standard deviation increased. A comparison of the NASBA results for both HPV 16 oligo sequences and SiHa cell lines showed that all parameters had the same trend for microsystems as well as for conventional methods except for the ratio between the levels of the fluorescence at the start and at the end of the amplification reaction. Background noise is more distinctive at small reaction chambers than for macroscopic fluorescence methods. Parts of the background fluorescence were removed from the assay by applying a thin gold layer on the backside of the polymer microchips. The COC itself is autofluorescent always giving some background fluorescence. Another contribution to noise detection is light scattering due to less perfect polymer surfaces. Time-to-positivity decreased for lower concentration as expected because the substrates used longer times to find and interact with the substrates. For the highest concentrations especially for the artificial HPV 16 in the experiments the time-to-positivity increase. Very high sample concentrations may also inhibit the reaction and therefore use longer time than an ideal reaction mixture. In the same manners the average slope decreases. When smaller amounts of target are in the reaction mixture to begin with, less amplicons will be produced and the slope will become lower than for higher concentrations. The detection limit of the NASBA reaction depends on the target of interest, the design of the primers and probe. In these experiments we were able to detect concentrations down to 1 pM and 20 cells/µl in both detection systems. Accordingly, this Example shows that it is possible to detect artificial HPV 16 sequences down to 1 pM concentration in polymer microchips utilizing real-time NASBA. For cell-line samples the detection limit were 20 cells/µl. These detection limits are the same that were obtained for experiments performed in the conventional Biotek reader.

**Table 1: NASBA performed on microchips detecting HPV 16 oligo sequences and SiHa cell-line dilution series. The results are the average and standard deviation of all values obtained in the experiments.**

| **Concentration** | **Ratio** | **Start point** | **Average slope** | **Positive amplifications / Number of reactions** | **Number of chips tested** |
|---|---|---|---|---|---|
| **HPV 16 oligo sequence [µM]** | | | | | |
| 0.1 | 2.90 ± 0.33 | 12.31 ± 5.36 | 45.09 ± 9.89 | 50 / 50 | 5 |
| 0.01 | 3.06 ± 0.37 | 14.73 ± 4.03 | 43.48 ± 9.48 | 40 / 40 | 4 |
| 0.001 | 2.65 ± 0.42 | 9.00 ± 2.05 | 45.99 ± 17.66 | 30 / 30 | 3 |
| 0.0001 | 2.75 ± 0.32 | 22.19 ± 4.45 | 35.08 ± 17.94 | 30 / 30 | 3 |
| 0.00001 | 2.56 ± 0.38 | 22.55 ± 7.36 | 29.87 ± 13.74 | 30 / 30 | 3 |
| 0.000001 | 2.54 ± 0.46 | 25.30 ± 3.60 | 19.62 ± 9.21 | 30 / 30 | 3 |
| 0.0000001 | 2.10 ± 0.32 | 37.09 ± 12.74 | 17.27 ± 11.78 | 33 / 70 | 7 |
| 0.00000001 | 1.85 ± 0.28 | 43.75 ± 7.13 | 9.94 ± 3.55 | 6 / 60 | 6 |
| 0.000000001 | 2.27 ± 0.86 | 81.00 ± 38.18 | 15.02 ± 6.26 | 2 / 60 | 6 |
| 0.0000000001 | 3.93 | 4.50 | 21.83 | 1 / 60 | 6 |
| **SiHa cell line [cells/**µ**l]** | | | | | |
| 2000 | 2.86 ± 0.30 | 16.91 ± 2.67 | 42.57 ± 6.24 | 40 / 40 | 4 |
| 200 | 2.80 ± 0.43 | 18.89 ± 3.39 | 40.56 ± 14.50 | 40 / 40 | 4 |
| 20 | 2.88 ± 0.27 | 30.65 ± 9.28 | 37.49 ± 11.09 | 39 / 40 | 4 |
| 2 | 2.75 ± 0.50 | 38.02 ± 26.12 | 35.09 ± 15.47 | 60 / 70 | 7 |
| 0.2 | 2.73 ± 0.54 | 70.13 ± 39.12 | 39.29 ± 14.97 | 4 / 50 | 5 |
| 0.02 | 0 | 0 | 0 | 0 / 30 | 3 |

**Table 2: Conventional NASBA testing performed on HPV 16 oligo sequences and SiHa cell-lines. The results are the average and standard deviation of all values obtained in the experiments.**

| **Concentration** | **Ratio** | **Start point** | **Average slope** | **Positive amplifications / Total reactions** |
|---|---|---|---|---|
| **HPV 16 oligo sequence [µM]** | | | | |
| 0.1 | 6.51 ± 0.18 | 14.00 ± 0.77 | 111.21 ± 19.29 | 6 / 6 |
| 0.01 | 6.74 ± 0.27 | 11.75 ± 1.47 | 96.26 ± 28.28 | 6 / 6 |
| 0.001 | 6.47 ± 0.28 | 15.25 ± 1.75 | 113.05 ± 33.62 | 6 / 6 |
| 0.0001 | 5.18 ± 1.07 | 23.83 ± 4.65 | 94.42 ± 58.85 | 6 / 6 |
| 0.00001 | 4.80 ± 1.17 | 25.13 ± 3.68 | 84.10 ± 38.27 | 12 / 12 |
| 0.000001 | 3.84 ± 0.81 | 26.25 ± 5.52 | 42.68 ± 11.40 | 12 / 12 |
| 0.0000001 | 1.79 ± 0.09 | 33.75 ± 7.42 | 15.71 ± 1.53 | 2/12 |
| 0.00000001 | - | - | - | 0 / 12 |
| 0.000000001 | - | - | - | 0 / 12 |
| 0.0000000001 | - | - | - | 0 / 12 |
| **SiHa cell line [cells/µl]** | | | | |
| 2000 | 4.85 ± 0.58 | 29.25 ± 1.25 | 80.09 ± 6.80 | 6 / 6 |
| 200 | 3.84 ±1.22 | 29.25 ± 4.00 | 52.47 ± 24.82 | 6 / 6 |
| 20 | 3.66 ± 1.15 | 33.30 ± 7.82 | 44.04 ± 16.82 | 5 / 6 |
| 2 | 2.96 ± 0.42 | 39.75 ± 1.06 | 27.95 ± 7.15 | 2 / 6 |
| 0.2 | - | - | - | 0 / 6 |
| 0.02 | - | - | - | 0 / 6 |

## Claims

1. An integrated lab-on-a-chip diagnostic system (1) for carrying out a sample preparation process on a fluid sample containing cells and/or particles, the system (1) comprising the following components formed on a common substrate:
(a) an inlet (5) for a fluid sample;
(b) a lysis unit (10) for lysis of cells and/or particles contained in the fluid sample;
(c) a nucleic acid extraction unit (15) for extraction of nucleic acids from the cells and/or particles contained in the fluid sample;
(d) a reservoir (20) containing a lysis fluid;
(e) a reservoir (25) containing an eluent for removing nucleic acids collected in the nucleic acid extraction unit (15);
wherein the sample inlet (5) is in fluid communication with the lysis unit (10), an optional valve (12) being present to control the flow of fluid therebetween;
wherein the lysis unit (10) is in fluid communication with the nucleic acid extraction unit (15), an optional valve (17) being present to control the flow of fluid therebetween;
wherein the reservoir (20) containing the lysis fluid is in fluid communication with the lysis unit (10), a valve (23) being present to control the flow of fluid therebetween;
wherein the reservoir (25) containing the eluent is in fluid communication with the nucleic acid extraction unit (15), a valve (28) being present to control the flow of fluid therebetween; and
wherein the system further comprises a single pump or syringe for actuation of all liquids.

2. A system (1) as claimed in claim 1, wherein the reservoir (20) containing the lysis fluid is in fluid communication with the inlet (5), an optional valve (22) being present to control the flow of fluid therebetween.

3. A system (1) as claimed in claim 1 or claim 2, wherein the reservoir (25) containing the eluent is in fluid communication with the inlet (5), an optional valve (27) being present to control the flow of fluid therebetween.

4. A system (1) as claimed in any one of claims 1 to 3, further comprising (g) a nucleic acid reaction unit (30), preferably a nucleic acid sequence amplification and detection unit, wherein the nucleic acid extraction unit (15) is in fluid communication with the nucleic acid reaction unit (30), an optional valve (32) being present to control the flow of fluid therebetween.

5. A system (1)as claimed in any one of claims 1 to 4, further comprising (h) a waste unit (35), wherein the waste unit (35) is in fluid communication with the lysis unit (10), an optional valve (37) being present to control the flow of fluid therebetween.

6. A system (1) as claimed in any one of claims 1 to 5, further comprising (i) a reservoir containing a washing solvent, preferably ethanol, which reservoir is in fluid communication with the nucleic acid extraction unit (15), an optional valve being present to control the flow of fluid therebetween.

7. A system (1) as claimed in any one of claims 1 to 6, further comprising (j) a reservoir containing a further washing solvent, preferably isopropanol, which reservoir is in fluid communication with the nucleic acid extraction unit (15), an optional valve being present to control the flow of fluid therebetween.

8. A system (1) as claimed in claim 6 or claim 7, wherein the reservoir (25) containing the eluent is in fluid communication with the reservoir containing the first washing solvent and/or the reservoir containing the second washing solvent.

9. A system (1) as claimed in claim 8, wherein the eluent, the first washing solvent and/or the second washing solvent are contained in a common reservoir.

10. A system (1) as claimed in claim 9, wherein the eluent, the first washing solvent and/or the second washing solvent are separated from one another in the common reservoir by a fluid, preferably air.

11. A system (1) as claimed in claim 9 or claim 10, wherein the common reservoir comprises a conduit in fluid communication with the inlet (5) and the lysis unit (10).

12. A system (1) as claimed in any one of claims 1 to 11, further comprising (k) means for introducing a fluid sample and/or air into the inlet (5), said mean preferably comprising a pump or a syringe.

13. A system (1) as claimed in any one of claims 1 to 11, further comprising a filtration unit, which unit is in fluid communication with the lysis unit (10).

14. A system (1) as claimed in claim 13, wherein the filtration unit comprises one or more of a dead-end filter, a cross-flow filter (eg micro-structured channels, porous hollow fibres or membranes), a gravity settler, a centrifuge, an acoustic cell filter, an optical trap, dielectrophoresis (DEP), electrophoresis, flow cytometry and adsorption based methods.

15. A system (1) as claimed in any one of claims 1 to 11, wherein the lysis unit (10) further comprises means to filter the fluid sample.

16. A system (1) as claimed in claim 15, wherein said means comprises one or more of a dead-end filter, a cross-flow filter (eg micro-structured channels, porous hollow fibres or membranes), a gravity settler, a centrifuge, an acoustic cell filter, an optical trap, dielectrophoresis (DEP), electrophoresis, flow cytometry and adsorption based methods.

17. A system (1) as claimed in any one of the preceding claims, wherein the system further comprises means for heating the contents of the lysis unit (10) and/or the nucleic acid extraction unit (15).

18. A system (1) as claimed in claim 17, wherein said mean comprises one or more Peltier elements located in or adjacent the lysis unit (10) and/or the nucleic acid extraction unit (15).

19. A system (1) as claimed in any one of the preceding claims, wherein the nucleic acid extraction unit (15) is at least partially filled with silica beads or particles.

20. A system (1) as claimed in claim 19, wherein the nucleic acid extraction unit (15) further comprises one or more sets of electrodes adjacent the silica beads or particles for collecting and/or preconcentrating the eluted nucleic acids.

21. A system (1) as claimed in claim 20, wherein said one or more sets of electrodes comprises platinum electrodes.

22. A system (1) as claimed in any one of the preceding claims for extracting nucleic acids present in a biological fluid, a dairy product, an environmental fluid or drinking water.

23. An apparatus for the analysis of biological and/or environmental samples, the apparatus comprising a system as defined in any one of the preceding claims.

24. An assay kit for the analysis of biological and/or environmental samples, the kit comprising a system as defined in an one of the claims 1 to 22 and means for contacting the sample with the system.

25. An apparatus as claimed in claim 23 or an assay kit as claimed in claim 24 which is disposable.

## Patentansprüche

1. Integriertes Lab-on-a-Chip-Diagnosesystem (1) zur Durchführung eines Probenaufbereitungsverfahrens einer flüssigen Probe, die Zellen und/oder Partikel enthält, wobei das System (1) die folgenden Bestandteile, auf einem gemeinsamen Substrat angeordnet, umfasst:
(a) einen Einlass (5) für eine flüssige Probe;
(b) eine Lyseeinheit (10) für die Lyse von Zellen und/oder Partikeln, die in der flüssigen Probe enthalten sind;
(c) eine Nukleinsäure-Extraktionseinheit (15) für die Extraktion von Nukleinsäuren aus den Zellen und/oder Partikeln, die in der flüssigen Probe enthalten sind;
(d) ein Reservoir (20), das ein Lysefluid enthält;
(e) ein Reservoir (25), das ein Elutionsmittel enthält, um die in der Nukleinsäure-Extraktionseinheit (15) gesammelten Nukleinsäuren zu entfernen;
wobei der Probeneinlass (5) in Fluidverbindung mit der Lyseeinheit (10) ist, wobei gegebenenfalls ein Ventil (12) vorhanden ist, um den Fluidstrom dazwischen zu kontrollieren;
wobei die Lyseeinheit (10) in Fluidverbindung mit der Nukleinsäure-Extraktionseinheit (15) ist, wobei gegebenenfalls ein Ventil (17) vorhanden ist, um den Fluidstrom dazwischen zu kontrollieren;
wobei das Reservoir (20), das das Lysefluid enthält, in Fluidverbindung mit der Lyseeinheit (10) ist, wobei ein Ventil (23) vorhanden ist, um den Fluidstrom dazwischen zu kontrollieren;
wobei das Reservoir (25), das das Elutionsmittel enthält, in Fluidverbindung mit der Nukleinsäure-Extraktionseinheit (15) ist, wobei ein Ventil (28) vorhanden ist, um den Fluidstrom dazwischen zu kontrollieren; und
wobei das System weiterhin eine einzelne Pumpe oder Spritze umfasst, um alle Flüssigkeiten anzutreiben.

2. System (1), wie in Anspruch 1 beansprucht, wobei das Reservoir (20), das das Lysefluid enthält, in Fluidverbindung mit dem Einlass (5) ist, wobei gegebenenfalls ein Ventil (22) vorhanden ist, um den Fluidstrom dazwischen zu kontrollieren.

3. System (1), wie in Anspruch 1 oder Anspruch 2 beansprucht, wobei das Reservoir (25), das das Elutionsmittel enthält, in Fluidverbindung mit dem Einlass (5) ist, wobei gegebenenfalls ein Ventil (27) vorhanden ist, um den Fluidstrom dazwischen zu kontrollieren.

4. System (1), wie in einem der Ansprüche 1 bis 3 beansprucht, weiterhin umfassend (g) eine Nukleinsäure-Reaktionseinheit (30), bevorzugt eine Nukleinsäuresequenz-Amplifikations- und -Detektionseinheit, wobei die Nukleinsäure-Extraktionseinheit (15) in Fluidverbindung mit der Nukleinsäure-Reaktionseinheit (30) ist, wobei gegebenenfalls ein Ventil (32) vorhanden ist, um den Fluidstrom dazwischen zu kontrollieren.

5. System (1), wie in einem der Ansprüche 1 bis 4 beansprucht, weiterhin umfassend (h) eine Abfalleinheit (35), wobei die Abfalleinheit (35) in Fluidverbindung mit der Lyseeinheit (10) ist, wobei gegebenenfalls ein Ventil (37) vorhanden ist, um den Fluidstrom dazwischen zu kontrollieren.

6. System (1), wie in einem der Ansprüche 1 bis 5 beansprucht, weiterhin umfassend (i) ein Reservoir, das ein Wasch-Lösungsmittel, bevorzugt Ethanol, enthält, wobei das Reservoir in Fluidverbindung mit der Nukleinsäure-Extraktionseinheit (15) ist, wobei gegebenenfalls ein Ventil vorhanden ist, um den Fluidstrom dazwischen zu kontrollieren.

7. System (1), wie in einem der Ansprüche 1 bis 6 beansprucht, weiterhin umfassend (j) ein Reservoir, das ein weiteres Wasch-Lösungsmittel, bevorzugt Isopropanol, enthält, wobei das Reservoir in Fluidverbindung mit der Nukleinsäure-Extraktionseinheit (15) ist, wobei gegebenenfalls ein Ventil vorhanden ist, um den Fluidstrom dazwischen zu kontrollieren.

8. System (1), wie in Anspruch 6 oder Anspruch 7 beansprucht, wobei das Reservoir (25), das das Elutionsmittel enthält, in Fluidverbindung mit dem Reservoir, das das erste Wasch-Lösungsmittel enthält, und/oder mit dem Reservoir, das das zweite Wasch-Lösungsmittel enthält, ist.

9. System (1), wie in Anspruch 8 beansprucht, wobei das Elutionsmittel, das erste Wasch-Lösungsmittel und/oder das zweite Wasch-Lösungsmittel in einem gemeinsamen Reservoir enthalten sind.

10. System (1), wie in Anspruch 9 beansprucht, wobei das Elutionsmittel, das erste Wasch-Lösungsmittel und/oder das zweite Wasch-Lösungsmittel in dem gemeinsamen Reservoir durch ein Fluid, bevorzugt Luft, voneinander getrennt sind.

11. System (1), wie in Anspruch 9 oder Anspruch 10 beansprucht, wobei das gemeinsame Reservoir eine Leitung in Fluidverbindung mit dem Einlass (5) und der Lyseeinheit (10) umfasst.

12. System (1), wie in einem der Ansprüche 1 bis 11 beansprucht, weiterhin umfassend (k) Mittel, um eine flüssige Probe und/oder Luft in den Einlass (5) einzuführen, wobei das Mittel bevorzugt eine Pumpe oder eine Spritze umfasst.

13. System (1), wie in einem der Ansprüche 1 bis 11 beansprucht, weiterhin umfassend eine Filtrationseinheit, wobei die Einheit in Fluidverbindung mit der Lyseeinheit (10) ist.

14. System (1), wie in Anspruch 13 beansprucht, wobei die Filtrationseinheit eines oder mehrere aus einem Dead-End-Filter, einem Querfluss-Filter (z. B. mikrostrukturierte Kanäle, poröse Hohlfasern oder Membranen), einem Schwereabscheider, einer Zentrifuge, einem akustischen Zellfilter, einer optischen Falle, Dielektrophorese (DEP), Elektrophorese, Durchflusszytometrie und absorptionsbasierten Verfahren umfasst.

15. System (1), wie in einem der Ansprüche 1 bis 11 beansprucht, wobei die Lyseeinheit (10) weiterhin ein Mittel umfasst, um die flüssige Probe zu filtrieren.

16. System (1), wie in Anspruch 15 beansprucht, wobei das Mittel eines oder mehrere aus einem Dead-End-Filter, einem Querfluss-Filter (z. B. mikrostrukturierte Kanäle, poröse Hohlfasern oder Membranen), einem Schwereabscheider, einer Zentrifuge, einem akustischen Zellfilter, einer optischen Falle, Dielektrophorese (DEP), Elektrophorese, Durchflusszytometrie und absorptionsbasierten Verfahren umfasst.

17. System (1), wie in einem der vorhergehenden Ansprüche beansprucht, wobei das System weiterhin ein Mittel zum Erwärmen des Inhalts der Lyseeinheit (10) und/oder der Nukleinsäure-Extraktionseinheit (15) umfasst.

18. System (1), wie in Anspruch 17 beansprucht, wobei das Mittel ein oder mehrere Peltier-Element(e) umfasst, das/die in oder neben der Lyseeinheit (10) und/oder der Nukleinsäure-Extraktionseinheit (15) angeordnet ist/sind.

19. System (1), wie in einem der vorhergehenden Ansprüche beansprucht, wobei die Nukleinsäure-Extraktionseinheit (15) zumindest teilweise mit Silica-Kügelchen oder -Partikeln gefüllt ist.

20. System (1), wie in Anspruch 19 beansprucht, wobei die Nukleinsäure-Extraktionseinheit (15) weiterhin einen oder mehrere Satz/Sätze von Elektroden neben den Silica-Kügelchen oder -Partikeln umfasst, um die eluierten Nukleinsäuren zu sammeln und/oder vorab einzuengen.

21. System (1), wie in Anspruch 20 beansprucht, wobei der eine oder die mehreren Satz/Sätze von Elektroden Platinelektroden umfasst/umfassen.

22. System (1), wie in einem der vorhergehenden Ansprüche beansprucht, zur Extraktion von Nukleinsäuren, die in einer biologischen Flüssigkeit, einem Molkereiprodukt, einer Flüssigkeit aus der Umwelt oder im Trinkwasser vorhanden sind.

23. Apparat für die Analyse von biologischen und/oder Umweltproben, wobei der Apparat ein System, wie in einem der vorhergehenden Ansprüche definiert, umfasst.

24. Assay-Kit für die Analyse von biologischen und/oder Umweltproben, wobei das Kit ein System, wie in einem der Ansprüche 1 bis 22 definiert, und Mittel, um die Probe mit dem System in Kontakt zu bringen, umfasst.

25. Apparat, wie in Anspruch 23 beansprucht, oder Assay-Kit, wie in Anspruch 24 beansprucht, der/das wegwerfbar ist.

## Revendications

1. Système de diagnostic intégré (1) laboratoire sur puce pour exécuter un processus de préparation d'échantillon sur un échantillon de fluide contenant des cellules et/ou des particules, le système (1) comprenant les composants suivants formés sur un substrat commun :
(a) une entrée (5) pour un échantillon de fluide ;
(b) une unité de lyse (10) pour lyser des cellules et/ou des particules contenues dans l'échantillon de fluide ;
(c) une unité (15) d'extraction d'acides nucléiques pour l'extraction d'acides nucléiques des cellules et/ou des particules contenues dans l'échantillon de fluide ;
(d) un réservoir (20) contenant un fluide de lyse ;
(e) un réservoir (25) contenant un éluant pour retirer les acides nucléiques collectés dans l'unité (15) d'extraction d'acides nucléiques ;
où l'entrée (5) pour échantillon est en communication fluidique avec l'unité de lyse (10), une soupape optionnelle (12) étant présente pour réguler l'écoulement de fluide entre les deux ;
où l'unité de lyse (10) est en communication fluidique avec l'unité (15) d'extraction d'acides nucléiques, une soupape optionnelle (17) étant présente pour réguler l'écoulement de fluide entre les deux ;
où le réservoir (20) contenant le fluide de lyse est en communication fluidique avec l'unité de lyse (10), une soupape (23) étant présente pour réguler l'écoulement de fluide entre les deux ;
où le réservoir (25) contenant l'éluant est en communication fluidique avec l'unité (15) d'extraction d'acides nucléiques, une soupape (28) étant présente pour réguler l'écoulement de fluide entre les deux ; et
où le système comprend en plus une seule pompe ou seringue pour l'actionnement de tous les liquides.

2. Système (1) tel que revendiqué dans la revendication 1, dans lequel le réservoir (20) contenant le fluide de lyse est en communication fluidique avec l'entrée (5), une soupape optionnelle (22) étant présente pour réguler l'écoulement de fluide entre les deux.

3. Système (1) tel que revendiqué dans la revendication 1 ou 2, dans lequel le réservoir (25) contenant l'éluant est en communication fluidique avec l'entrée (5), une soupape optionnelle (27) étant présente pour réguler l'écoulement de fluide entre les deux.

4. Système (1) tel que revendiqué dans l'une quelconque des revendications 1 à 3, comprenant en plus (g) une unité (30) de réaction d'acides nucléiques, préférablement une unité de détection et d'amplification de séquences d'acides nucléiques, où l'unité (15) d'extraction d'acides nucléiques est en communication fluidique avec l'unité (30) de réaction d'acides nucléiques, une soupape optionnelle (32) étant présente pour réguler l'écoulement de fluide entre les deux.

5. Système (1) tel que revendiqué dans l'une quelconque des revendications 1 à 4, comprenant en plus (h) une unité de rejet (35), dans lequel l'unité de rejet (35) est en communication fluidique avec l'unité de lyse (10), une soupape optionnelle (37) étant présente pour réguler l'écoulement de fluide entre les deux.

6. Système (1) tel que revendiqué dans l'une quelconque des revendications 1 à 5, comprenant en plus (i) un réservoir contenant un solvant de lavage, préférablement de l'éthanol, lequel réservoir est en communication fluidique avec l'unité (15) d'extraction d'acides nucléiques, une soupape optionnelle étant présente pour réguler l'écoulement de fluide entre les deux.

7. Système (1) tel que revendiqué dans l'une quelconque des revendications 1 à 6, comprenant en plus (j) un réservoir contenant un solvant de lavage supplémentaire, préférablement de l'isopropanol, lequel réservoir est en communication fluidique avec l'unité (15) d'extraction d'acides nucléiques, une soupape optionnelle étant présente pour réguler l'écoulement de fluide entre les deux.

8. Système (1) tel que revendiqué dans la revendication 6 ou 7, dans lequel le réservoir (25) contenant l'éluant est en communication fluidique avec le réservoir contenant le premier solvant de lavage et/ou le réservoir contenant le deuxième solvant de lavage.

9. Système (1) tel que revendiqué dans la revendication 8, dans lequel l'éluant, le premier solvant de lavage et/ou le deuxième solvant de lavage sont contenus dans un réservoir commun.

10. Système (1) tel que revendiqué dans la revendication 9, dans lequel l'éluant, le premier solvant de lavage et/ou le deuxième solvant de lavage sont séparés l'un de l'autre dans le réservoir commun par un fluide, préférablement de l'air.

11. Système (1) tel que revendiqué dans la revendication 9 ou 10, dans lequel le réservoir commun comprend un conduit en communication fluidique avec l'entrée (5) et l'unité de lyse (10).

12. Système (1) tel que revendiqué dans l'une quelconque des revendications 1 à 11, comprenant en plus (k) un moyen pour introduire un échantillon de fluide et/ou de l'air dans l'entrée (5), ledit moyen comprenant préférablement une pompe ou une seringue.

13. Système (1) tel que revendiqué dans l'une quelconque des revendications 1 à 11, comprenant en plus une unité de filtration, laquelle unité est en communication fluidique avec l'unité de lyse (10).

14. Système (1) tel que revendiqué dans la revendication 13, dans lequel l'unité de filtration comprend un ou plusieurs d'un filtre à bout mort, d'un filtre à écoulements croisés (par exemple, canaux micro-structurés, membranes ou fibres creuses poreuses), d'un décanteur par gravité, d'une centrifugeuse, d'un filtre à cellules acoustiques, d'un piège optique, d'une diélectrophorèse (DEP), d'une électrophorèse, d'une cytométrie en flux, et des procédés basés sur l'absorption.

15. Système (1) tel que revendiqué dans l'une quelconque des revendications 1 à 11, dans lequel l'unité de lyse (10) comprend en plus un moyen pour filtrer l'échantillon de fluide.

16. Système (1) tel que revendiqué dans la revendication 15, dans lequel ledit moyen comprend un ou plusieurs d'un filtre à bout mort, d'un filtre à écoulements croisés (par exemple, canaux micro-structurés, membranes ou fibres creuses poreuses), d'un décanteur par gravité, d'une centrifugeuse, d'un filtre à cellules acoustiques, d'un piège optique, d'une diélectrophorèse (DEP), d'une électrophorèse, d'une cytométrie en flux, et des procédés basés sur l'absorption.

17. Système (1) tel que revendiqué dans l'une quelconque des revendications précédentes, dans lequel le système comprend en plus un moyen pour chauffer le contenu de l'unité de lyse (10) et/ou de l'unité (15) d'extraction d'acides nucléiques.

18. Système (1) tel que revendiqué dans la revendication 17, dans lequel ledit moyen comprend un ou plusieurs éléments Peltier situés dans ou de manière adjacente à l'unité de lyse (10) et/ou à l'unité (15) d'extraction d'acides nucléiques.

19. Système (1) tel que revendiqué dans l'une quelconque des revendications précédentes, dans lequel l'unité (15) d'extraction d'acides nucléiques est au moins partiellement remplie de particules ou de perles de silice.

20. Système (1) tel que revendiqué dans la revendication 19, dans lequel l'unité (15) d'extraction d'acides nucléiques comprend en plus un ou plusieurs ensembles d'électrodes adjacentes aux particules ou perles de silice pour collecter et/ou pré-concentrer les acides nucléiques élués.

21. Système (1) tel que revendiqué dans la revendication 20, dans lequel ledit un ou plusieurs ensembles d'électrodes comprend des électrodes en platine.

22. Système (1) tel que revendiqué dans l'une quelconque des revendications précédentes pour extraire des acides nucléiques présents dans un fluide biologique, un produit laitier, un fluide environnemental ou de l'eau potable.

23. Appareil pour l'analyse d'échantillons biologiques et/ou environnementaux, l'appareil comprenant un système selon l'une quelconque des revendications précédentes.

24. Kit d'essai pour l'analyse d'échantillons biologiques et/ou environnementaux, le kit comprenant un système selon l'une quelconque des revendications 1 à 22 et un moyen pour mettre l'échantillon en contact avec le système.

25. Appareil selon la revendication 23 ou kit d'essai selon la revendication 24 qui est jetable.
